Europäisches Patentamt

European Patent Office

Office européen des brevets

(11) Publication number: **0 229 830 B1**

# EUROPEAN PATENT SPECIFICATION

(45) Date of publication of patent specification: **09.11.94**

(51) Int. Cl.5: **A61K 41/00, A61M 1/36**

(21) Application number: **86904617.7**

(22) Date of filing: **02.07.86**

(86) International application number:
**PCT/US86/01409**

(87) International publication number:
**WO 87/00053 (15.01.87 87/01)**

The file contains technical information submitted
after the application was filed and not included in
this specification

(54) **METHOD OF REDUCING IMMUNOGENICITY AND INDUCING IMMUNOLOGIC TOLERANCE.**

(30) Priority: **05.07.85 US 752452**

(43) Date of publication of application:
**29.07.87 Bulletin 87/31**

(45) Publication of the grant of the patent:
**09.11.94 Bulletin 94/45**

(84) Designated Contracting States:
**DE FR GB IT**

(56) References cited:
**EP-A- 0 138 489**     **WO-A-85/00954**
**FR-A- 975 851**       **US-A- 397 001**
**US-A- 3 683 183**     **US-A- 4 456 589**
**US-A- 4 576 143**

**See also references of WO8700053**

(73) Proprietor: **Puget Sound Blood Center and Program**
**921 Terry Avenue**

Seattle, Washington 98104-1256 (US)

Proprietor: **Fred Hutchinson Cancer Research Center**
**1124 Columbia Street**
**Seattle Washington 98104 (US)**

(72) Inventor: **SLICHTER, Sherill J.**
**P.O.Box 903**
**Vashon Island, Washington 98070 (US)**
Inventor: **DEEG, H. Joachim**
**203 Euclid Avenue**
**Seattle, Washington 98122 (US)**

(74) Representative: **Brown, John David et al**
**FORRESTER & BOEHMERT**
**Franz-Joseph-Strasse 38**
**D-80801 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention relates to a method of reducing the immunogenicity of allogeneic tissues such as blood transfusions and allografts, and to methods of inducing immunologic tolerance to allogeneic tissues in putative recipients.

Background Art

Patients with a variety of disorders receive intermittent or chronic transfusion support, or require tissue grafting to replace a defective organ. Individuals requiring transfusion support have either a genetic or acquired deficiency of one or more blood components that require replacement therapy. Many different products prepared from blood are available for transfusion, including both cellular and plasma components. However, repeated exposure to blood products often results in recipient recognition of the foreign transfused antigens. Such immune recognition of foreign antigens results in a failure to achieve a benefit from the transfusion, and in some circumstances may even cause a transfusion reaction with adverse consequences to the recipient.

Several approaches have been used to either prevent or delay alloimmunization. The majority of these techniques involve giving immunosuppressive therapy to the transfusion recipient to prevent recognition of the transfused foreign antigens. Such immunosuppressive therapy is often inadequate to suppress the recognition process, resulting in alloimmunization in spite of the treatment. Furthermore, the immunosuppressive therapy may have undesirable side effects, including organ toxicity and immunosuppression of desirable responses, such as recognition and destruction of pathogenic bacteria.

Once an immune response to foreign antigens has occurred, there is little evidence that any immunosuppressive therapy is beneficial. Continued adequate transfusion support is possible only if antigen matching between donor and recipient is achieved. Often a matched donor is not available, or for some transfusion products so little is known about the antigen systems involved in the immune response that laboratory methods are not available to appropriately select a matched donor.

An alternative approach to preventing alloimmunization, other than immunosuppressing the recipient, is to reduce the immunogenicity of the transfused product. As all transfused blood products are immunogenic and will eventually induce an immune response in most transfused recipients, any procedure that can prevent or at least delay immunization is beneficial. Selecting only antigen compatible donors beginning with the first transfusion is possible in some circumstances, but for the majority of patients either not enough donors are available to continue this process or a matching procedure does not exist.

For organ grafting, because there is persistent exposure to foreign tissue antigens, eventual rejection of the grafted tissue occurs. To prevent graft rejection several approaches have been used: recipient immunosuppression, matching tissue antigens of donor and recipient, reducing the immunogenicity of the grafted tissue, or inducing in the recipient a state of tolerance to the foreign antigens of the graft. Furthermore, depending on the tissue being grafted, different approaches may be required to achieve a successful graft, and combined therapies may be additive in their beneficial effects. For example, in bone marrow transplantation massive doses of chemo-radiotherapy are given to the recipient to destroy the recipient's autologous marrow and to induce immunosuppression to allow engraftment of the donor marrow. Even better results are obtained if marrow donor and recipient are related and well-matched for the major histocompatibility antigen system (HLA). Although post-marrow grafting immunosuppression is usually performed, it is for only a limited time.

In contrast, for kidney grafting lesser degrees of immunosuppressive therapy are required to avoid unacceptable marrow and gastrointestinal toxicity. Furthermore, continuous post-grafting immunosuppression is required. Often a related kidney donor is not available, and lesser degrees of HLA matching between donor and recipient are more often accepted than for bone marrow transplantation.

Another major difference between these two types of tissue grafting is the effect of prior transfusions on engraftment. For kidney graft recipients, prior transfusions, particularly from the intended kidney donor, are beneficial, apparently by inducing some degree of tolerance to the subsequent kidney graft. However, prior blood transfusions before marrow grafting, especially if the blood has come from the intended marrow donor, markedly increase the risk of graft rejection. Thus, although there are similarities in procedures to enhance organ grafts (immunosuppression and donor-recipient HLA matching), there are clear differences in (1) the amount, type, and duration of immunosuppression required; (2) the acceptance of non-HLA identity between organ donor and recipient; and (3) the effect of prior transfusions on enhancing or impairing a subsequent organ graft. Furthermore, even the best combined therapies are not always successful in ensuring a successful organ graft, and there may be substantial toxicities associated with the therapies being used.

Besides using HLA matching and recipient immunosuppression, efforts to directly reduce immunogenicity of the engrafted tissue or to induce tolerance in the recipient, other than by prior blood transfusion in key recipients, have been limited. In bone marrow transplantation, efforts to purify or enrich the marrow graft for stem cells, and to eliminate T-lymphocytes that may be responsible for graft-vs.-host disease (a post-grafting complication), have often resulted in a transplanted marrow that has failed to engraft. Some investigators have stored or cultured the graft in vitro prior to transplantation (skin grafting) to facilitate engraftment. Most of these latter methods to enhance organ grafting have had limited success.

WO 85/00954 relates to enhancement of a patients' acceptance of transplanted tissue by irradiating donor-specific blood diluted 1:50 with phosphate-buffered saline, with a high dose of irradiation, transfusing the diluted, irradiated blood into the patient and then transplanting the tissue. FR-A-0975851 relates to apparatus for irradiating infected blood with ultra-violet light to destroy viral infection. EP-A-0138489 relates to the treatment of excessive lymphocyte populations by the treatment of whole blood with a psoralen capable of forming adducts with DNA in the presence of ultra-violet light.

It would be advantageous to avoid immune recognition by the recipient of incompatible donor antigens and the consequent destruction of allogeneic tissue following transfusion or transplantation.

## Disclosure of the Invention

Briefly stated, there are disclosed (a) methods for reducing the immunogenicity of blood products and transfused or transplanted tissue; and (b) methods and compositions for substantially reducing sensitization of a recipient to subsequently introduced blood products, transfused or transplanted tissue. Compositions of blood products or transfused or transplanted tissue for use as active therapeutic substances are also disclosed.

A method is disclosed for reducing the immunogenicity of blood products. Suitable blood products include red cells, white cells, platelets, and plasma constituents. Plasma constituents include, for example, albumin, clotting factor proteins, gamma globulin and antithrombin-III. The method generally involves exposing the blood products to a source of UV irradiation sufficient to reduce the immunogenicity of the blood products. Generally, the UV irradiation results in an exposure of from 120 $J/m^2$ to 13,500 $J/m^2$.

According to the present invention there is provided a method of obtaining a viable blood product for use in a replacement therapy, characterized by reducing the immogenicity of viable blood products in vitro, by exposing a blood product to a source of UV irradiation resulting in an exposure of from 120 $J/m^2$ to 13,500 $J/m^2$.

In one embodiment of this method, the dose of UV irradiation does not substantially interfere with the viability or physiological function of the blood products. The threshold exposure to UV irradiation, at which substantial interference with viability or physiological function occurs, con be determined by reference to identifiable parameters of viability and function for unmanipulated, non-UV irradiated tissue using standard laboratory testing protocols. Where the blood product is platelets, for instance, the UV irradiation results in an exposure of from 120 $J/m^2$ to 360 $J/m^2$. Where the blood product is red blood cells, one specific embodiment comprises UV irradiation resulting in an exposure of approximately 13,500 $J/m^2$.

There is disclosed a method for reducing the immunogenicity of transfused or transplanted tissue in vitro. Suitable transfused or transplanted tissues include tissues such as bone marrow, skin, bone, and cornea, and organs including heart, lung, liver, pancreas and kidney. The method generally involves exposing the transfused or transplanted tissue to a source of UV irradiation sufficient to reduce the immunogenicity of the transfused or transplanted tissue.

Within this method, generally the UV irradiation results in an exposure of from 120 $J/m^2$ to 13,500 $J/m^2$. In one embodiment of this method, the dose of UV irradiation does not substantially interfere with the viability or physiological function of the transfused or transplanted tissue. As noted above, the threshold exposure to the UV irradiation at which substantial interference with viability or physiological function occurs can be determined by using standard laboratory testing protocols. Where the transfused or transplanted tissue is pancreatic cells, for instance, the UV irradiation results in an exposure that should not exceed 9,000 $J/m^2$.

There is further disclosed a series of compositions useful as active therapeutic substances. These compositions include blood products, or transfused or transplanted tissue exposed to a source of UV irradiation sufficient to reduce the immunogenicity of the blood products, transfused or transplanted tissue. Another group of compositions includes blood products and transfused or transplanted tissue exposed to a source of UV irradiation sufficient to substantially reduce the sensitization of a recipient to subsequently introduced blood products, transfused or transplanted tissue. As described below, these compositions have

EP 0 229 830 B1

a variety of uses as active therapeutic substances.

Other aspects of the present invention will become evident upon reference to the following detailed description.

Best Mode for Carrying Out the Invention

The transfusion of whole blood or blood products may be given to patients who are deficient in one or more substances that are present in whole blood. Patients requiring transfusions may have either a congenital or acquired deficiency of the substance being replaced by transfusion. The patient's deficiency may occur for a limited period of time or may be chronic. For those individuals with a chronic deficiency of a component of whole blood, continued beneficial effects of the transfusion are dependent upon the recipient not developing an immune response to the transfused material. For come transfused substances, the antigenic nature of the product is well-defined and long-term compatibility between donor and recipient is often achieved. For example, the major antigens on the surface of red cells that need to be matched between donor and recipient are the A and B antigens and the RhD antigens. The gene frequency of these antigens in the population makes it relatively easy to ensure finding a compatible donor for almost all recipients. In contrast, platelets express HLA antigens on their surface as the major alloantigenic system that must be matched between donor and recipient to ensure compatibility. However, the genetic heterogeneity of the HLA system makes matching of this complex antigen system between donor and recipient difficult. Thus, patients requiring chronic platelet transfusion support often become alloimmunized to HLA antigens that are different between donor and recipient. In addition, there are platelet-specific antigens which also may need to be matched between donor and recipient. Thus, the development of alloantibodies to donor platelets often occurs and is difficult to manage.

Similarly, organ and tissue transplants generally require antigenic compatibility between donor and recipient for the HLA antigen system as well as the ABO system. Furthermore, in spite of compatibility for all the recognized blood group and histocompatibility antigen systems, continued functioning of the transplanted organ or tissue requires that temporary or permanent immunosuppressive therapy be provided to the recipient. Often, either the degree of antigenic compatibility between donor and recipient and/or the effectiveness of the immunosuppressive therapy given to the recipient is not sufficient to prevent graft rejection. Because the transplanted organ or tissue is often vital to the well-being of the recipient, failure of engraftment may result in substantial recipient morbidity or mortality.

Thus, any procedure which can reduce the immunogenicity of the transfused or transplanted tissue or, alternatively, reduce sensitization to the subsequent introduction of these tissues, may result in a substantial benefit to the recipient. One of the purposes of this invention is to provide a method of reducing immunogenicity or reducing sensitization, so that individuals who need replacement of either transfused or transplanted tissue can have a successful outcome. In addition, it is believed that the techniques used to reduce immunogenicity and reduce sensitization ore sufficiently efficacious that the requirement for antigenic compatibility between donor and recipient, in order to ensure a successful transfusion or transplantation procedure, is obviated.

The immunogenicity of the transfused or transplanted tissue is reduced by directly exposing the tissue to ultraviolet (UV) irradiation prior to administering the tissue to the recipient. Alternatively, a state of tolerance in the recipient to non-UV or UV-exposed allogeneic tissue or organs may be induced by prior exposure to UV-irradiated allogeneic tissue.

In the first situation, where the objective is to reduce the immunogenicity of the transfused or transplanted tissue or organ, it is important to determine that the UV-exposure does not substantially interfere with the viability or physiologic function of the allogeneic tissue. The threshold exposure of UV-irradiation, at which substantial interference with viability or physiologic function occurs, can be determined by reference to tissue-specific parameters of viability and function for unmanipulated, non-UV-irradiated tissue or organs, using standard laboratory testing protocols. (Lindahl-Kissling, K. and J. Safwenberg, Int. Arch. Allergy 41: 670, 1971; Dutcher et al., Blood 58: 1007, 1981; Weiden et al., J. Immunol. 117: 143, 1976; Granstein et al., J. Immunol. 132: 2210, 1984).

For some transplanted tissues, a UV dose effective in reducing immunogenicity may be impossible to administer due to the bulk nature of the engrafting tissue, e.g., cardiac and kidney grafts, recognizing the low penetrance of UV-irradiation. Alternatively, for some tissues, the threshold exposure at which biologic activity is substantially unimpaired may be too low to prevent immune recognition when the tissue is administered to the recipient. Many experimental and clinical studies have attempted to change the immunoresponse of the prospective transplant recipient by transfusing normal blood products prior to transplantation. Although this approach has proven beneficial in many instances (i.e., graft survival was

4

EP 0 229 830 B1

improved), it was detrimental in others because the recipient became sensitized and the subsequent grafts were rejected in an accelerated fashion. UV-exposed blood may have the same beneficial effect as normal blood and, in addition, may not lead to sensitization. The term sensitization, as used in the present invention, refers to the presence of antibodies or lymphocytes in the recipient that are either directed against or capable of recognizing donor material. This recognition will normally result in destruction of the transfused product or rejection of the graft. For these circumstances, a UV-irradiated tissue which allows acceptance of the graft can be administered to the recipient prior to or concurrent with the administration of the transfused or transplanted tissue, such as an organ.

For the purpose of reducing sensitization or inducing tolerance, the dose of UV-irradiation need not necessarily maintain the biologic activity of the UV-irradiated blood product or tissue being used to reduce sensitization or induce tolerance. There may be be no requirement for the tissue which allows acceptance of the graft or blood product to be derived from the same individual as the one from whom the subsequent transfusion or graft is obtained, nor is there even a requirement that they be derived from a related or histologically identical individual. In fact, as outlined in Table 1, the donor of the UV-exposed blood product or tissue may be either related or unrelated to the recipient of the transfused or transplanted tissue or blood product and, furthermore, may not be required to be antigenically matched with the recipient of the transfused and/or transplanted tissue or blood product.

## TABLE 1

| Donor | Recipient |
|---|---|
| | UV-Exposed or Normal |
| UV-Exposed Blood | Transfused/Transplanted |
| Products or Tissue | Tissue or Blood Products |
| **Blood Products** | |
| Whole blood, red blood cells, white blood cells, platelets and plasma constituents (albumin, clotting factors, gamma globulin, antithrombin-III, etc.) | **Organs** (e.g., heart, lung, liver, pancreas, kidney, etc.) **Tissue** (e.g., bone marrow, skin, cornea, bone, etc.) |
| **Tissue** (e.g., bone marrow, skin, cornea, bone, etc., | **Blood Products** Whole blood, red blood cells, white blood cells, platelets and plasma constituents (albumin, clotting factors, gamma globulin, antithrombin-III, etc.) |

Relationship Between Donor of UV-Exposed Blood Product

and

Transfused/Transplanted Recipient

Autologous or Syngeneic

Related -- Antigenically matched, or mismatched

Unrelated -- Antigenically matched, or mismatched

To summarize the principles of this invention, three illustrative examples are provided. Transfusions of normal blood from the intended donor prior to bone marrow transplantation will result in sensitization of the recipient in all instances, as determined by marrow graft rejection. If, however, blood products are UV irradiated prior to transfusion, sensitization can be substantially, if not entirely, eliminated, as shown in Example 1, by successful bone marrow engraftment. Thus, recipients given UV irradiated blood, in contrast to recipients given normal blood products, behave similarly to recipients who have not been given a transfusion. In the illustration, the donor of the blood and bone marrow was a DLA-matched littermate of the transfused and transplanted recipient.

In the second example, the process of reducing the immunogenicity of a transfused blood component (platelets), while maintaining viability and function of the UV-exposed material to allow for effective platelet support, is detailed. The effectiveness of the procedure in reducing the immunogenicity of the transfused material is illustrated by the fact that the procedure was successful in preventing alloimmune platelet destruction between randomly selected unrelated donor and recipient pairs.

In addition, the latter studies provide evidence that in at least a portion of the recipients, a state of unresponsiveness (non-specific tolerance) was induced by the transfusion of UV-irradiated blood products, since subsequent transfusion of normal blood products did not result in sensitization.

The following examples are offered by way of illustration, and not by way of limitation.

Comparative EXAMPLE 1

In these experiments using a canine model, it is demonstrated that exposure of whole blood to ultraviolet light before transfusion prevents immunization and allows for subsequent marrow engraftment.

Normal dogs given 9.2 Gy of total body irradiation and bone marrow transplants from DLA (major histocompatibility complex) - identical littermate donors generally achieve sustained engraftment and become long-term survivors. However, if recipient dogs are transfused with whole blood from the marrow donor before transplantation, the marrow graft is always rejected and the recipient dogs uniformly die with marrow aplasia. Such graft rejections are apparently the result of transfusion-induced sensitization of the recipient against minor (non-DLA) histocompatibility antigens of the donor.

In this Example, normal dogs were given 9.2 Gy total body irradiation, hemopoietic marrow infusion from DLA-identical littermates, and post-transplant conditioning as described in Blood 54:477, 1979. Prior to marrow grafting, recipients were given 3 transfusions, on days 24, 17, and 10 before transplantation. The transfusions consisted of either unmanipulated, UV-irradiated or sham-irradiated whole blood from the marrow donor. The following procedure was used. Whole blood (50 ml/transfusion) was obtained from a donor Beagle dog by venipuncture with syringes containing preservative-free heparin to prevent coagulation. The blood was then diluted 1:1.5 with Waymouth's Minimal Medium. Aliquots of 7.5 ml were placed in 10 plastic dishes (Falcon #3003), resulting in a layer of 1.5 mm thickness in each dish. The uncovered plates, placed on a shaker platform to assure continual mixing of the blood, were then exposed for 30 minutes to UV irradiation (220-300 nm wavelength) from a germicidal lamp (General Electric) at an intensity of 750 uW/cm$^2$ (total exposure 1.35 mJ/cm$^2$), as determined with a Black Ray shortwave UV meter (U.V. Products). Cells were then quantitatively recovered from the dishes, collected into syringes and injected into a recipient dog. Sham-irradiated blood was handled in an identical fashion, except that it was exposed to visible light instead of UV light.

Table 2 shows the results of these experiments.

TABLE 2

| Group | Transfusions | No. of Dogs | | | Incidence Sustained Graft |
|---|---|---|---|---|---|
| | | Studied | With Graft Rejection | With Sustained Graft | |
| 6 | None | 60 | 1 | 59 | 98% |
| 7 | Whole blood unmanipulated | 21 | 21 | 0 | 0% |
| 8 | Whole blood, UV-irradiated | 10 | 0 | 10 | 100% |
| 9 | Whole blood, sham-irradiated | 4 | 4 | 0 | 0% |

The majority of the dogs in Groups 6 and 7 were included in the above-cited previous report. Of the ten dogs in Group 8 that had a sustained graft, three died with septicemia at 8, 9 and 13 days post-grafting, respectively. Engraftment was documented by rising granulocyte counts following the post-irradiation nadir, and by the presence of erythroid and myeloid precursor cells (total cellularity 10-25% of normal) on marrow samples obtained at autopsy. The other seven dogs are surviving after more than 100 days; in these dogs sustained engraftment was documented by the presence of the donor sex karyotype in all metaphase spreads from bone marrow and peripheral blood (four dogs) or by conversion to the donor erythrocyte antigen pattern (three dogs). Dogs in group 9 died on days 10, 11, 11, and 13, respectively, with septicemia. Narrow cellularity at autopsy was less than 5% in all of these dogs. Cells were composed of plasma cells and reticulum cells; hemopoietic precursor cells were absent.

All of the dogs that were given transfusions of UV-exposed blood prior to marrow transplantation sustained engraftment and behaved like dogs not given any transfusions before transplantation. In contrast, all of the control dogs, transfused before transplantation with blood exposed to light in the visible range, rather than the UV range, failed to achieve substantial engraftment and died with bone marrow aplasia, similar to the dogs given transfusions of unmanipulated whole blood. Thus, exposure of blood to UV light appears to abrogate the sensitizing ability of blood, and allows for subsequent successful engraftment.

In patients undergoing marrow transplantation for various diseases, especially severe aplastic anemia, exposure to transfusion products before marrow transplantation, as in our dog model, leads to a significant risk of subsequent marrow graft rejection. While patients who are untransfused at the time of marrow transplantation usually achieve sustained engraftment and become healthy survivors, patients who have been given transfusions before transplantation have been reported in previous studies to have an incidence of graft rejection as high as 60%. Since graft rejection is usually not compatible with the patient's survival, it is highly desirable to prevent sensitization of the patient before transplantation. The method described herein provides such an approach. Furthermore, it is contemplated that this method may result in tolerance of other transplants, e.g., kidney, heart, etc., as well.

EXAMPLE 2

Platelet transfusion experiments were performed between unrelated donor-recipient dog pairs that were randomly selected from a pool of animals. The experimental design involved preparing a platelet concentrate from 50 ml of blood drawn from a donor animal, and radiolabelling the donor's platelets with $^{51}$Chromium as described by Weiden et al., J. Immunol. 117:143-150, 1976. After transfusion of the radiolabelled donor dog's platelets into a recipient, serial blood samples were drawn from the recipient to determine the disappearance rate of the donor's platelets from the recipient's circulation. If only 5% or less of the radiolabelled donor platelets were circulating in the recipient at 24 hours post-transfusion, the animal was considered to be alloimmunized to the donor's platelets.

In 21 recipients, weekly transfusion of a single, unrelated donor's platelets resulted in immunization in 20 recipients (95%) after an average of 3.1 ± 0.7 transfusions (range 2-12). Of these non-irradiated control transfusion recipients, 18/21 (86%) were immunized by ≦ 8 donor transfusions.

In an effort to prevent immune recognition of the transfused platelets, 12 randomly-selected, unrelated donor-recipient pairs were transfused with UV-irradiated platelets. Before these transfusion studies were begun, 14 animals had their autologous platelets UV-irradiated, and after radiolabelling these platelets were returned to the donor. These experiments showed that UV-irradiation doses of greater than 600 $\mu$watts/cm$^2$ for more than 10 minutes (total dose of 3,600 J/m$^2$) resulted in loss of platelet viability, as documented by an absent or reduced autologous platelet survival determination. Based on these autologous studies, for the experimental paired donor-recipient transfusion studies doses of UV irradiation that did not produce loss of platelet viability were selected and used.

Specifically, 12 recipient animals received $^{51}$Cr radiolabelled donor platelets that had been exposed to 200, 400, or 600 $\mu$watts of UV irradiation/cm$^2$ for one minute, delivered by a germicidal lamp (220-300 nm wavelength) (total dose of 120, 240, and 360 J/m$^2$, respectively). The platelets were exposed to the UV-irradiation by placing them at a depth of about 1-2 cm in an open Petri dish which was continually agitated throughout the UV exposure. The same randomly-selected donor-recipient pairs were used for a maximum of eight weekly transfusions or until alloimmunization occurred. Only 1/12 (8%) of the recipient dogs who received UV-irradiated donor platelets became immunized.

Next, in the 11 non-immunized recipients (after the eight weeks of UV-irradiated, unrelated donor platelets had been given), an additional 8 weeks of non-UV-irradiated platelet transfusions from the same donor were given. Only 3/11 (27%) of these recipients were able to recognize the non-UV-irradiated platelets from their unrelated donor. In addition, if non-UV-irradiated platelets from a new unrelated donor were subsequently given to the 8 recipients who had failed to recognize non-UV-irradiated platelets from their original donor, 10/23 (43%) of these donors failed to cause alloimmunization in the recipient even after 8 transfusions. The other 3 were immunized by the new donor's platelets by 1, 1 and 9 transfusions, respectively. These data demonstrate that tolerance to non-UV-irradiated transfused donor platelets was induced in these recipients by prior transfusions of UV-irradiated platelets, and that the tolerance was not specific for the original donor's platelets.

EXAMPLE 3

UV exposure as described in comparative Example 1 is compatible with useful red cell transfusion support. In one dog the survival of UV-exposed -$^{51}$Chromium labelled red blood cells was measured according to standard techniques. The T$_{\frac{1}{2}}$ was 17 days, which compares to a range of 17-24 days measured in dogs given unmanipulated transfusions.

8

EP 0 229 830 B1

**Claims**

1. A method of obtaining a viable blood product for use in a replacement therapy, characterized by reducing the immogenicity of viable blood products in vitro, by exposing a blood product to a source of UV irradiation resulting in an exposure of from 120 J/m$^2$ to 13,500 J/m$^2$.

2. The method of Claim 1 wherein the blood product is selected from the group consisting of red cells, white cells, platelets and plasma constituents.

3. The method of Claim 1 or 2 wherein the blood product is platelets and the UV irradiation results in an exposure of from 120 J/m$^2$ to 360 J/m$^2$.

4. The method of Claim 1 or 2 wherein the blood product is red blood cells and the UV irradiation results in an exposure of 13,500 J/m$^2$.

**Patentansprüche**

1. Ein verfahren zum Erhalten eines lebensfähigen Blutproduktes zur Verwendung in einer Substitutionstherapie, gekennzeichnet durch das Reduzieren der Immunogenizität lebensfähiger Blutprodukte in vitro, indem ein Blutprodukt einer UV-Bestrahlungsquelle ausgesetzt wird, was zu einer Strahlendosis von 120 J/m$^2$ bis 13.500 J/m$^2$ führt.

2. Das verfahren von Anspruch 1, wobei das Blutprodukt aus der Gruppe ausgewählt ist, die aus roten Blutzellen, weißen Blutzellen, Blutplättchen und Plasmabestandteilen besteht.

3. Das verfahren von Anspruch 1 oder 2, wobei das Blutprodukt Blutplättchen ist und die UV-Bestrahlung zu einer Strahlendosis von 120 J/m$^2$ bis 360 J/m$^2$ führt.

4. Das Verfahren von Anspruch 1 oder 2, wobei das Blutprodukt rote Blutzellen ist und die UV-Bestrahlung zu einer Strahlendosis von 13.500 J/m$^2$ führt.

**Revendications**

1. Procédé d'obtention d'un produit sanguin viable destiné à être utilisé dans un traitement substitutif, caractérisé par la réduction de l'immunogénicité de produits sanguins viables in vitro, par exposition d'un produit sanguin à une source de rayonnement UV, ayant pour résultat une exposition de 120 J/m$^2$ à 13 500 J/m$^2$.

2. Procédé suivant la revendication 1, dans lequel le produit sanguin est choisi dans le groupe consistant en globules rouges, globules blancs, plaquettes et constituants plasmatiques.

3. Procédé suivant la revendication 1 ou 2, dans lequel le produit sanguin consiste en plaquettes et l'irradiation avec un rayonnement UV a pour résultat une exposition de 120 J/m$^2$ à 360 J/m$^2$.

4. Procédé suivant la revendication 1 ou 2, dans lequel le produit sanguin consiste en globules rouges et l'irradiation avec un rayonnement UV a pour résultat une exposition de 13 500 J/m$^2$.

9